# EUROPÄISCHE PATENTSCHRIFT

(11) **EP 2 126 540 B1**
(45) Veröffentlichungstag und Bekanntmachung des Hinweises auf die Patenterteilung: **09.04.2014**
(21) Anmeldenummer: 08706886.2
(22) Anmeldetag: 13.02.2008
(51) Int. Cl.: G01N 1/28

(54) **BEGASUNGSVORRICHTUNG UND -SYSTEM**
FUMIGATION DEVICE AND SYSTEM
DISPOSITIF ET SYSTÈME DE CONDITIONNEMENT SOUS GAZ

(30) Priorität: 21.02.2007 DE 202007002538 U
(43) Veröffentlichungstag der Anmeldung: 02.12.2009
(73) Patentinhaber: Pieter Van Weenen&Co. Gmbh, 79183 Waldkirch (DE)
(72) Erfinder: KREBS, Tobias, 79261 Gutach (DE)
(74) Vertreter: von Pichler, Cletus
(86) Internationale Anmeldenummer: PCT/DE2008/000271
(87) Internationale Veröffentlichungsnummer: WO 2008/101471

(56) Entgegenhaltungen:
- WO-A-02/063027
- WO-A-2004/076609
- DE-A1- 10 329 983
- DE-A1- 19 712 575
- DE-A1-102006 043 656
- US-A- 4 201 845
- US-A- 5 766 556

## Beschreibung

### Gebiet der Erfindung

Die vorliegende Erfindung betrifft im Allgemeinen die Begasung von Proben und insbesondere eine Begasungsvorrichtung sowie ein wenigstens eine solche umfassendes System.

### Hintergrund der Erfindung

Zur Untersuchung von Proben ist es bekannt, diese mit Medium zu begasen. Dies wird beispielsweise bei Zellkulturen verwendet, um deren Eigenschaften bei Exposition gegenüber einer oder mehreren gasförmigen Stoffen zu analysieren.

Die bei Begasung üblicherweise verwendeten Vorrichtungen umfassen Behälter, in denen zu untersuchende Proben angeordnet werden. Den Probenbehältern kann zur Begasung der Proben vorgesehenes Medium zugeführt werden. Das in die Probenbehälter eingebrachte Begasungsmedium tritt in Kontakt mit der jeweilige Probe.

Bekannte Ansätze verwenden komplexe, ortsfeste Anordnungen mit zum Teil zahlreichen empfindlichen beispielsweise aus Glas hergestellten Komponenten. Die Herstellung und Änderungen solcher Anordnungen sind zeit-, kosten- und personalintensiv.

Anordnungen, die beispielsweise Begasungen von Proben im großen Maßstab und nach industriellen Qualitäts- und Kostenkriterien erlauben, fehlen.

Die DE 103 29 983 A1 offenbart eine Vorrichtung mit den Merkmalen des Oberbegriffs des Anspruchs 1. Die WO 2004/076609 A1, DE 197 12 575 A1 und WO 02/063027 A1 offenbaren Vorrichtungen mit einer Versorgungseinrichtung für Probenaufnahmen, um den Probenaufnahmen gasförmiges oder flüssiges Medium zuzuführen, und mit Mitteln zur Zufuhr von Medium für jede der Probenaufnahmen.

### Aufgabe der Erfindung

Aufgabe der vorliegenden Erfindung ist es, Lösungen bereitzustellen, die kompakt und variabel aufgebaute Anordnungen zur Begasung von Proben ermöglichen.

### Kurzbeschreibung der Erfindung

Zur Lösung der obigen Aufgabe stellt die vorliegende Erfindung eine Vorrichtung sowie ein System gemäß den unabhängigen Ansprüchen bereit.

Insbesondere stellt die vorliegende Erfindung wie im Anspruch 1 definiert, eine Begasungsvorrichtung mit einem einstückigen Probenaufnahmenblock und einem Begasungskopf bereit.

Der Probenaufnahmeblock umfasst wenigstens zwei darin ausgebildete, baueinheitlich integrierte Probenaufnahmen und zur Versorgung derselben mit Fluid eine ebenfalls darin ausgebildete, baueinheitlich integrierte Fluidversorgungseinrichtung.

Der Begasungskopf ist vorgesehen, um Begasungsmedium den Probenaufnahmen zuzuführen und ist unter Anderem hierfür mit dem Probenaufnahmenblock verbindbar. Der Begasungskopf umfasst für jede Probenaufnahme einen Auslass für Begasungsmedium und eine im Begasungskopf baueinheitlich integrierte, darin ausgebildete Einrichtung, um Begasungsmedium zu den Begasungsmediumauslässen zu führen. Die Begasungsmediumführung kann beispielsweise einen oder mehrere Kanäle, Leitungen oder andere Komponenten umfassen, mit denen Begasungsmedium befördert werden kann.

Die Versorgungseinrichtung kann zur separaten, individuellen Fluidversorgung für eine oder mehrere Probenaufnahmen ausgeführt sein.

Die Einrichtung zur Begasungsmediumführung zu den Probenaufnahmen kann einzelne oder mehrere Begasungsmediumauslässe separat mit Begasungsmedium versorgt werden.

Die Begasungsvorrichtung kann für wenigstens eine Probenaufnahme, vorzugsweise für alle Probenaufnahmen, eine individuelle Niveauerfassungseinrichtung aufweisen. Mit der Niveauabfassungseinrichtung kann das Niveau von in einer Probenaufnahme vorhandenem Fluid ermittelt werden.

Um einzelne, mehrere oder alle Probenaufnahmen individuell gesteuert mit Fluid zu versorgen, kann für entsprechende Probenaufnahmen jeweils ein steuerbares Schließglied verwendet werden, das mit dem zentralen Fluidkanal verbunden ist und Fluidzufuhr und/oder -abfuhr von bzw. zu diesem steuern kann.

Zur separaten Fluidversorgung einzelner, mehrerer oder aller Probenaufnahmen kann für jede dafür vorgesehene Probenaufnahme wenigstens ein separater Fluidkanal im Probenaufnahmenblock ausgebildet sein, der beispielsweise über eine Öffnung im Boden einer Probenaufnahme mit dieser verbunden ist. Fluidanschlüsse zur Zufuhr und/oder -abfuhr von Fluid, die mit separaten Fluidkanälen verbunden sind, können an Längsseiten des Probenaufnahmenblocks angeordnet sein.

Die Niveauerfassungseinrichtung kann für eine, mehrere oder alle Probenaufnahmen jeweils eine individuelle Niveaumesseinrichtung und/oder Niveauanzeige umfassen.

Im Fall einer individuellen Niveaumesseinrichtung kann diese an und/oder in einer jeweiligen Innenwand einer entsprechenden Probenaufnahme angeordnet sein. Individuelle Niveaumesseinrichtungen können einzelne, mehrere Sensoren, Detektoren und dergleichen umfassen, die elektrisch, elektronisch, optisch und/oder akustisch Fluidniveaus erfassen können.

Im Fall einer oder mehrerer individueller Niveauanzeigen können in einer Außenseite des Probenaufnahmeblocks über Durchgangsöffnungen mit Probenaufnahmen in Fluidverbindung stehende Kontrollkammer verwendet werden. Insbesondere ist es vorgesehen, die Kontrollkammern und deren Fluidverbindung(en) zu entsprechenden Probenaufnahmen so zu gestalten, dass ein in der Kontrollkammer vorhandenes Fluidniveau Aussagen über das Fluidniveau in der zugeordneten Probeaufnahme erlaubt oder dieses wiedergibt.

Zur optischen Kontrolle durch einen Benutzer können die Kontrollkammern jeweils einzeln, zu mehreren oder gemeinsam durchsichtig abgedeckt sein.

Zur Führung von Begasungsmedium zu Begasungsmediumauslässen können sich durch den Begasungskopf erstreckende Begasungsmediumkanäle vorhanden sein.

Die Begasungsmediumkanäle können sich jeweils zwischen einem Einlassanschluss zur Zufuhr von Begasungsmedium und einem Auslassanschluss erstrecken, über den Begasungsmedium entfernt werden kann.

Zur individuellen, separaten Steuerung von einzelnen, mehreren oder allen Begasungsmediumauslässen zur Verfügung stehendem Begasungsmedium können die entsprechenden Begasungsmediumauslässe über ein steuerbares Schließglied mit dem jeweiligen Begasungsmediumkanal verbindbar sein.

Der Begasungskopf kann für jeden einzelnen Begasungsmediumauslass eine separate Unterdruckkammer aufweisen. Ferner ist es vorgesehen, für mehrere oder alle Begasungsmediumauslässe eine gemeinsame Unterdruckkammer zu verwenden.

Die Unterdruckkammern stehen, im zusammengebauten Zustand, jeweils über wenigstens eine Ansaugöffnung mit einer entsprechenden Probenaufnahme in Verbindung.

Die Begasungsmediumauslässe können Auslassenden aufweisen, die beispielsweise hohlzylindrisch sind, **kegelstumpfförmige** Innenräume begrenzen, mehrere im Querschnitt jeweils mehreckige, vorzugsweise sechseckige Auslasskanäle umfassen und/oder mehrere im Querschnitt jeweils kreisförmige Auslasskanäle umfassen.

In Bereichen, in denen einzelnen, mehreren oder allen Begasungsmediumauslässen Begasungsmedium zugeführt wird, kann ein oder mehrere Fotometer angeordnet sein.

Der Probenaufnahmenblock kann einen thermisch leitenden Bodenbereich aufweisen, der ferner so gestaltet sein kann, dass er eine form- und/oder kraftschlüssige Wirkverbindung mit einer Basis ermöglicht.

Der Probenaufnahmeblock und der Vergasungskopf können lösbar und beispielsweise mittels eines Klemmmechanismus miteinander verbindbar sein.

Der Klemmmechanismus kann einen oder mehrere Hebel umfassen, mit dem bzw. denen der Probenaufnahmeblock und der Begasungskopf aneinander gesichert werden können. Der der die Hebel können drehbar an dem Probenaufnahmenblock angeordnet sein.

Des weiteren stellt die vorliegende Erfindung insbesondere ein Begasungssystem bereit, das wenigstens eine erfindungsgemäße Begasungsvorrichtung und eine Basis umfasst.

Die Basis kann eine Aufnahme aufweisen, in der ein Bodenbereich eines Probenaufnahmeblocks angeordnet werden kann.

Zur Wärmesteuerung der wenigstens einen Begasungsvorrichtung kann die Basis eine Wärmeabgabeeinrichtung umfassen, die beispielsweise unter Verwendung erwärmten Fluids und/oder elektrisch erwärmbarer Komponenten Wärme abgibt.

Weitere Ausführungsformen sind in den abhängigen Ansprüchen definiert und in der folgenden detaillierten Beschreibung von Ausführungsformen angegeben.

### Kurzbeschreibung der Zeichnungen

Bevorzugte Ausführungsformen der vorliegenden Erfindung werden im Folgenden unter Bezugnahme auf die beigefügten Zeichnungen erläutert, die zeigen:
- Fig. 1 bis 12: schematische Darstellungen von Ausführungsformen erfindungsgemäßer Begasungssysteme und Begasungsvorrichtungen,
- Fig. 13 und 14: schematische Darstellungen einer Ausführungsform einer erfindungsgemäßen Vorrichtung zum Mischen und Zuführen von Begasungsmedien; und
- Fig. 15: eine schematische Darstellung eines vorgesehenen Probenbehälters.

Für gleiche oder vergleichbare Komponenten sind unabhängig von den jeweiligen Ausführungsformen gleiche Bezugszeichen und gleiche Bezeichnungen verwendet.

Bezugszeichen sind nicht in allen Figuren wiederholt.

Dichtelemente, Dichtungen etc. sind - soweit nicht anders bezeichnet - mit "D" angegeben.

### Beschreibung bevorzugter Ausführungsformen

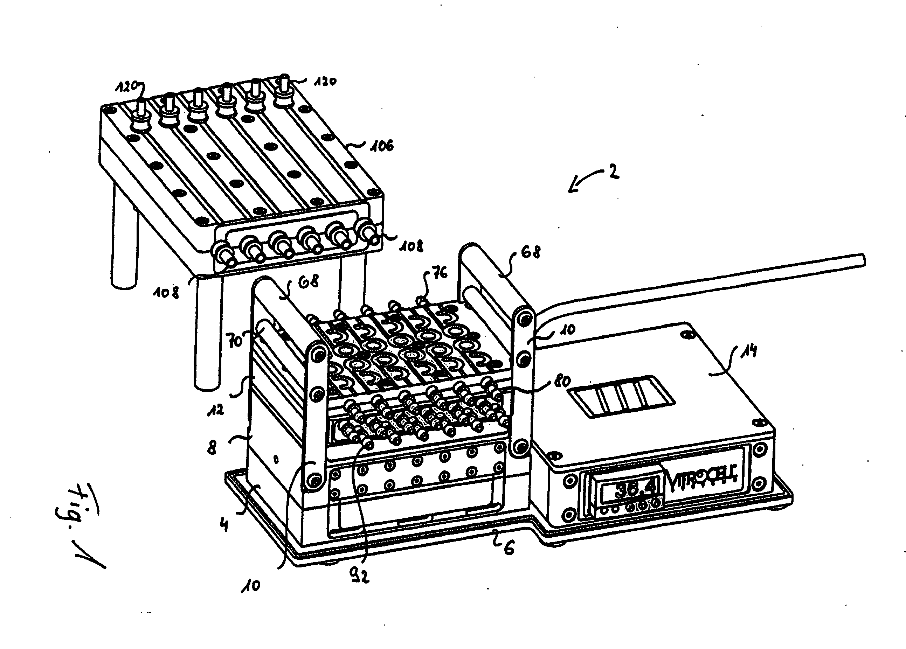

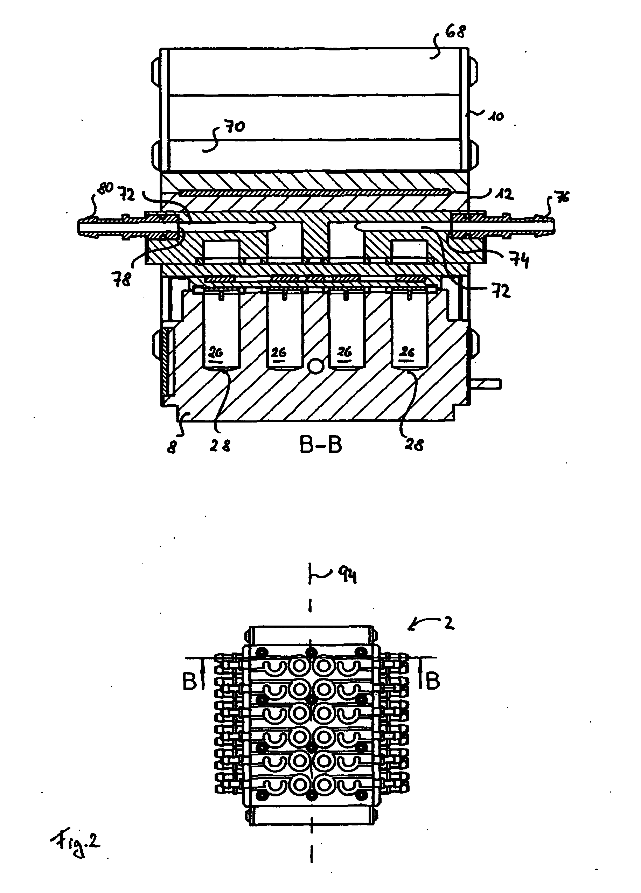

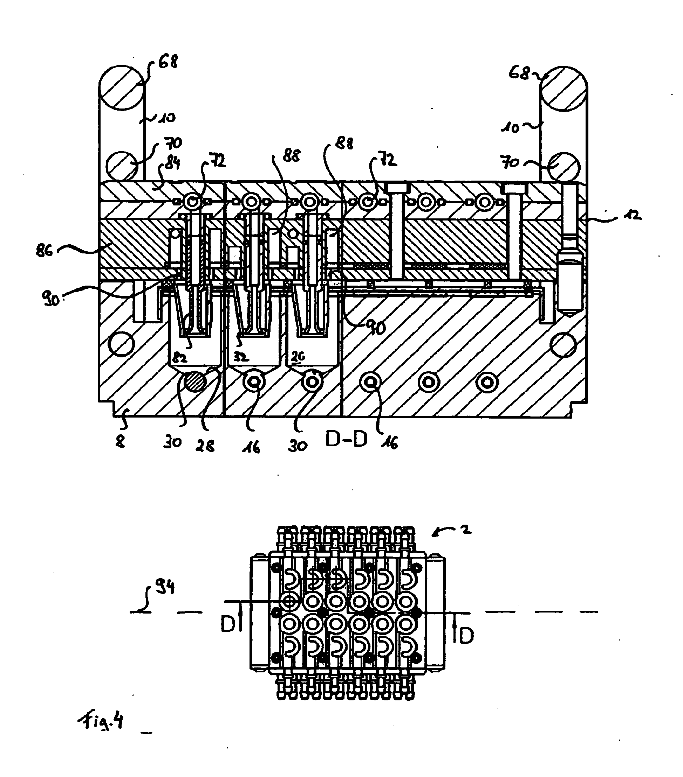

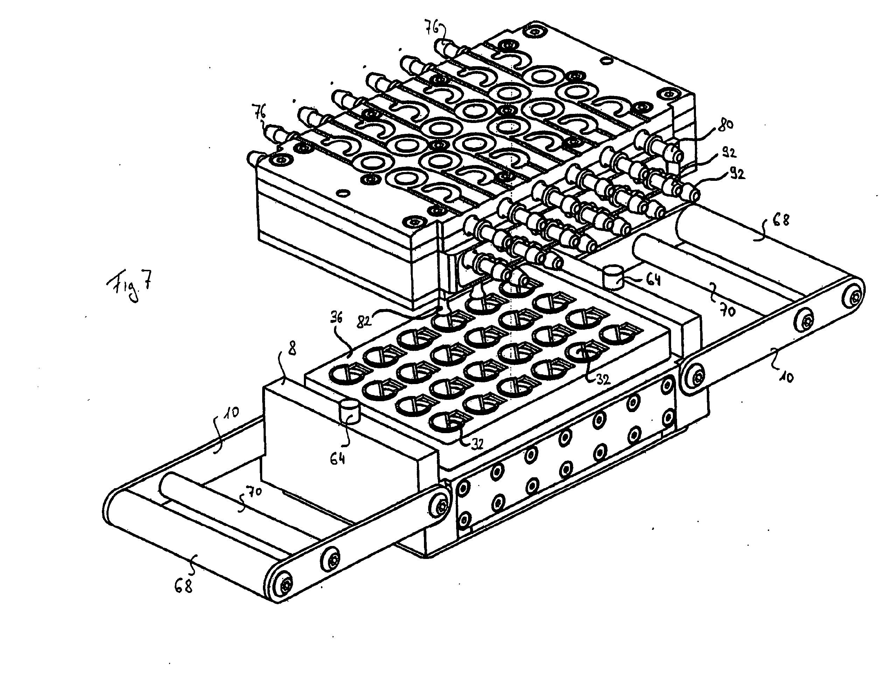

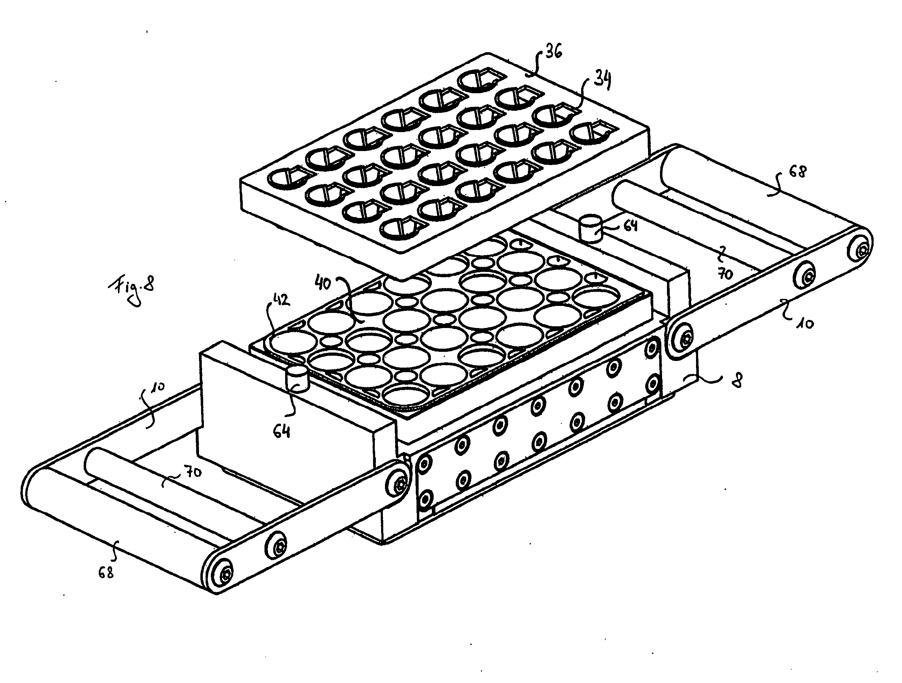

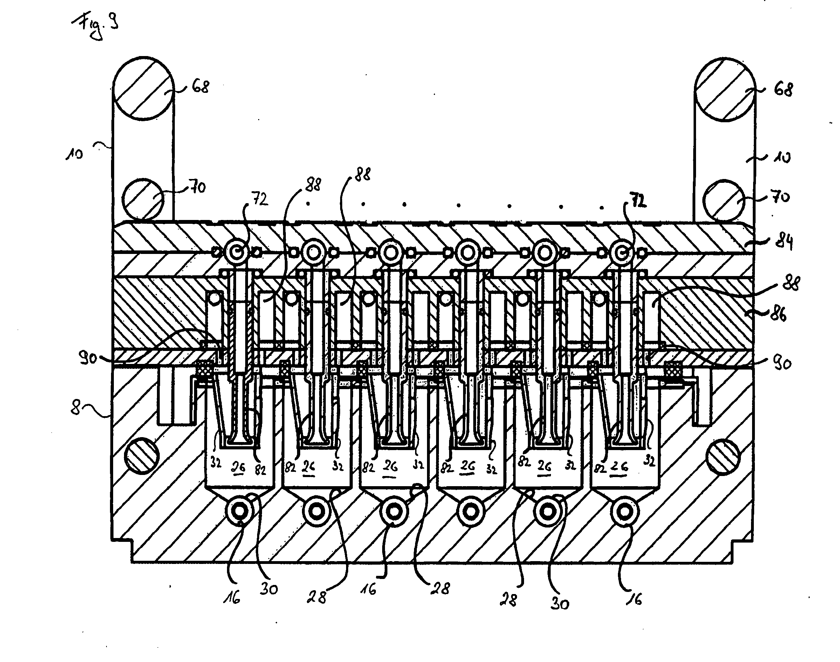

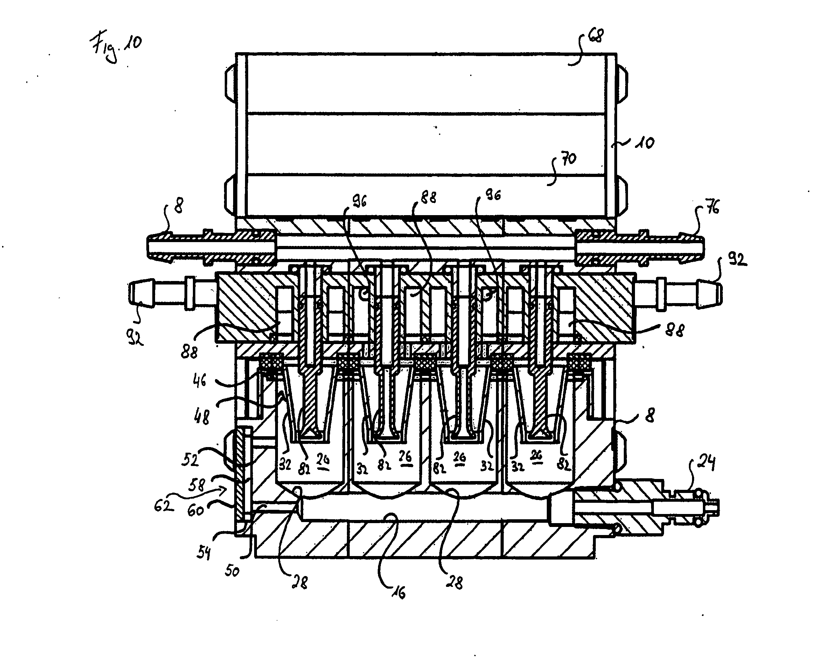

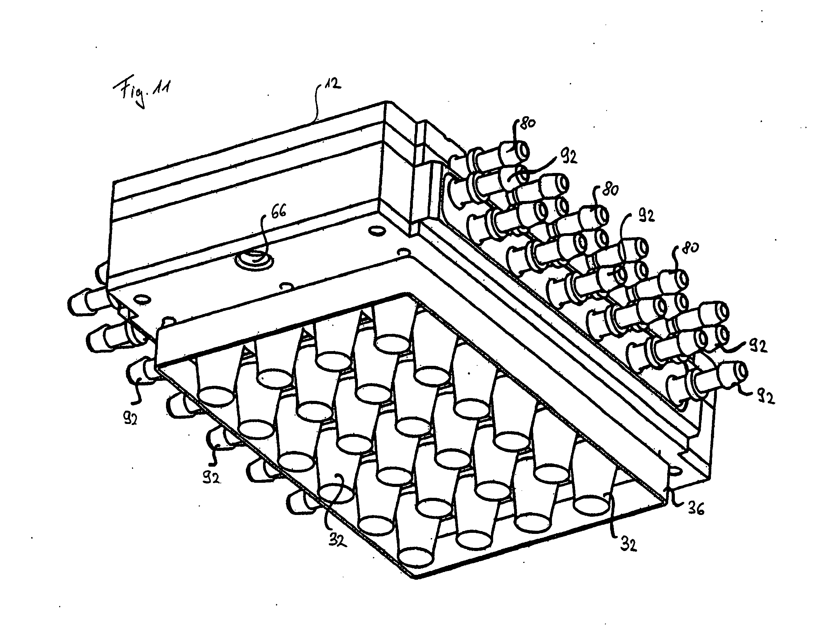

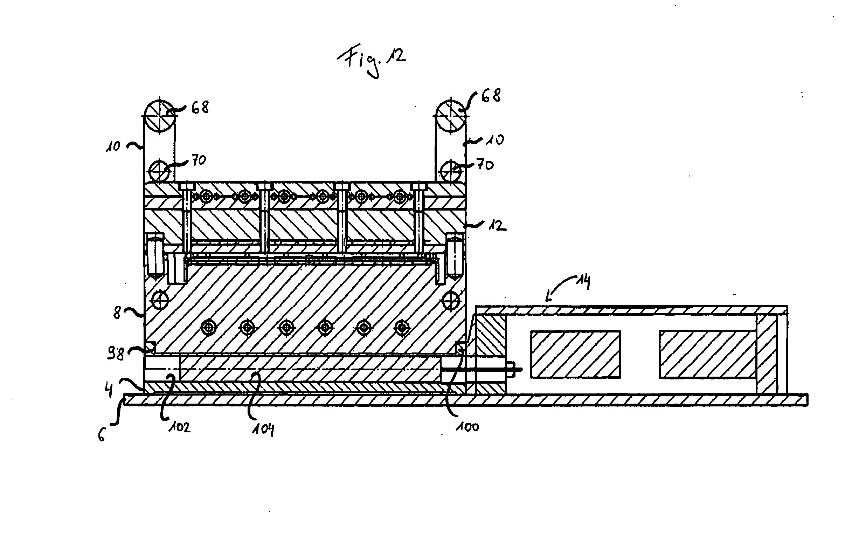

Bezugnehmend auf Fig. 1 bis 15 sind bevorzugte Ausführungsformen eines Begasungssystems und bevorzugte Ausführungsformen von damit verwendbaren bzw. davon umfassten Komponenten beschrieben.

Das im Ganzen mit 2 bezeichnete Begasungssystem umfasst eine Basis 4, die auf einer Fläche 6 (z. B. Tischoberfläche, Grundplatte etc.) angeordnet ist. Auf der Basis 4 ist ein Probenaufnahmenblock 8 angeordnet, der mittels zweier am Probenaufnahmenblock 8 schwenkbar angeordneten Hebeln 10 mit einem Begasungskopf 12 lösbar verbunden ist. Ferner ist eine Steuerung 14 vorgesehen.

Der Probenaufnahmenblock 8 und der Begasungskopf 12 bilden - zusammen mit weiteren Komponenten - eine Begasungsvorrichtung.

Der Probenaufnahmenblock 8 weist mehrere sich figurengemäß quer zu dessen Längsrichtung erstreckende zentrale Fluidkanäle 16 auf; bei nicht gezeigten Ausführungsformen erstrecken sich die Fluidkanäle 16 in Längsrichtung des Probenaufnahmeblocks 8. Darstellungsgemäß sind die Fluidkanäle 16 jeweils an einem Ende 18 verschlossen und an dem gegenüberliegenden Ende 22 mit einem aus dem Probenaufnahmeblock 8 ragenden Fluidanschluss 24 verbunden. Über die Fluidanschlüsse 24 können den Fluidkanälen 16 jeweils Fluid zugeführt und von diesen entfernt werden.

Alternativ können die Enden 18 der Fluidkanäle 16 jeweils offen und mit einem aus dem Probenaufnahmeblock 8 ragenden weiteren Fluidanschluss (nicht gezeigt) verbunden sein. Diese Ausführung ermöglicht es, Fluid durch die Fluidkanäle 16 (in beide Richtungen) zu befördern.

Der Probenaufnahmenblock 8 umfasst Probenaufnahmen 26. Die dargestellten Ausführungsformen umfassen beispielhaft 24 Probenaufnahme 26. Es können jedoch z.B. anwendungsabhängige andere Anzahlen Probenaufnahmen 26 verwendet werden. Die Probenaufnahmen 26 sind in den einstückigen Probenaufnahmenblock 8 eingearbeitet, beispielsweise durch Bohren und/oder Drehen, und/oder werden bei der Herstellung des Probenaufnahmenblocks 8 ausgebildet. Letzteres kann beispielsweise durch Verwendung von entsprechenden Gieß-, Spritz- oder Gussformen erfolgen.

Die Probenaufnahmen 26 weisen an ihren Böden 28 jeweils eine Öffnung 30 auf. Die Öffnungen 30 stehen in Fluidverbindung mit einem darunter liegenden der Fluidkanäle 16. Hierfür können, wie dargestellt, die Öffnungen 30 unmittelbar in den jeweiligen Fluidkanal 16 münden oder über nicht gezeigte Verbindungsstücke damit verbunden sein.

Jeder Fluidkanal 16 ist mit Probenaufnahmen 26 verbunden, die vertikal darüber angeordnet sind. Darstellungsgemäß sind dies die Probenaufnahmen 26, die in einer Reihe quer zur Längsrichtung der Probenaufnahmenblocks 8 angeordnet sind; bei nicht gezeigten Ausführungsformen können dies in einer Reihe in Längsrichtung angeordnete Probenaufnahmen 26 sein.

Die Fluidkanäle 16 dienen zur Versorgung der jeweils zugeordneten Probenaufnahmen 26 und darin angeordneter Proben mit Fluid.

In Probenaufnahmen 26 können unmittelbar, d. h. insbesondere ohne Verwendung zusätzlicher in Probenaufnahmen einsetzbare Komponenten, Proben (z. B. biologisches Gewebe, Zellkulturen, Bakterienkulturen etc.) angeordnet werden. Proben können auch unter Verwendung von Probenbehältern 32 in Probenaufnahmen 26 angeordnet werden. Beispiele für mögliche Probenbehälter umfassen sogenannte "Zellkultur-Membraneinsätze". Zur Anordnung von Probenbehälter können einzelne, mehrere oder alle Probenaufnahmen 26 mit einem Absatz 34 ausgebildet sein, an den sich beispielsweise ein Wulst oder Ring an einem Außenumfang eines Probenbehälters abstützen kann. Nähere Ausführungen hierzu finden sich unter Bezugnahme auf Fig. 25.

Insbesondere ist es vorgesehen, einen Einsatz 36 zu verwenden, der baueinheitlich integriert eine Anzahl an Probenbehältern 32 aufweist, die der Anzahl an Probenaufnahmen 26 entsprechen oder kleiner sein kann. Im letzteren Fall kann der Einsatz 36, dort wo kein Probenbehälter vorgesehen ist, eine Öffnung aufweisen, um die darunter liegenden Probenaufnahmen nutzen zu können, oder geschlossen sein, wodurch die darunter liegende Probenaufnahme abgedeckt und/oder abgedichtet sein kann und/oder nicht nutzbar sein kann.

Zur genauen Ausrichtung des Einsatzes 36 und der Probenaufnahmen 26 zu einander können an der Unterseite des Einsatzes 36 Nasen 38 ausgebildet sein, die in entsprechende Ausnehmungen (nicht bezeichnet) an den oberen offenen Enden der Probenaufnahmen 26 eingreifen können.

Zwischen dem Einsatz 36 und dem Probenaufnahmenblock 8 ist eine Dichtung 40 angeordnet, die - wie dargestellt - einstückig sein kann oder mehrere einzelne Dichtungselemente (nicht gezeigt) umfassen kann. Die Dichtung 40 weist einen darstellungsgemäß nach oben erhöhten, umlaufenden äußeren Rand 42 auf. Der Rand 42 kann z.B. innen gestuft und/oder schräg verlaufend ausgebildet sein, um den Einsatz 36 auszurichten und/oder Einsätze unterschiedlicher Abmessungen verwenden zu können.

Zwischen den Probenaufnahmen 26 und einer Seitenfläche 44 des Probenaufnahmenblocks 8 erstrecken sich jeweils erste Durchgangsöffnungen 46. Die ersten Durchgangsöffnungen 46 münden in oberen Bereichen der Probenaufnahmen 26 in diese benachbart zu den jeweiligen darstellungsgemäß oberen Probenaufnahmenöffnungen 48.

Über zweite Durchgangsöffnungen 50 stehen die Probenaufnahmen 26 ebenfalls mit der Seitenfläche 44 in Fluidverbindung. Gemäß den gezeigten Ausführungen erstrecken sich diese Fluidverbindungen zwischen der Seitenfläche 44, den zweiten Durchgangsöffnungen 50, dem jeweiligen Fluidkanal 16 und den Öffnungen 30 und den darstellungsgemäß unteren Bereichen der Probenaufnahmen 26. Bei nicht gezeigten Ausführungsformen ist es vorgesehen, dass zweite Durchgangsöffnung 50 unmittelbar in Bodenbereiche von Probenaufnahmen 26 münden.

Bei nicht gezeigten Ausführungsformen sind an oder vor den Öffnungen 30 (oder weiter unten beschriebenen vergleichbaren Öffnungen) von einzelnen, mehreren oder allen Probenaufnahmen 26 Schließglieder angeordnet. Vorgesehene Schließglieder umfassen Klappen, Ventile etc. mit offenen und geschlossenen Stellungen und/oder zum graduellen Öffnen und Schließen. Dies gilt auch für im Weiteren genannte Schließglieder. Dies ermöglicht eine individuelle Steuerung der Zufuhr und Abfuhr von Fluid zu bzw. von einzelnen, mehreren oder allen Probenaufnahmen 26.

Die Seitenfläche 44 ist in einer Außenseite 52 des Probenaufnahmenblocks 8 ausgebildeten Ausnehmung 54 bereitgestellt. In der Ausnehmung 54 ist beispielsweise mittels Schrauben, Nieten, Klebverbindungen und dergleichen ein Abstandselement 56 befestigt. Das Abstandselement 56 weist Fenster 58, Aussparungen, Ausschnitte oder vergleichbare materialfreie Besprechenden ersten und zweiten Durchgangsöffnungen 46 und 50 in den von den Fenstern 58 freigelassen Bereichen der Seitenfläche 44 enden.

Die Fenster 58 sind mit einer sich im Wesentlichen über das gesamte Abstandselement 56 erstreckende, durchsichtige Abdeckung 60 bedeckt. Alternativ können separate, über jedem Fenster 58 angeordnete Abdeckelemente verwendet werden.

Jedes Fenster 58 definiert zusammen mit der Abdeckung 60 und einem entsprechenden Bereich der Seitenfläche 44 eine Kontrollkammer 62, die über die jeweiligen ersten und zweiten Durchgangsöffnungen 46 und 50 mit einer entsprechenden Probenaufnahme 26 in Fluidverbindung stehen. Abgesehen von den ersten und zweiten Durchgangsöffnungen 46 und 50 ist es vorgesehen, dass die Kontrollkammer 62 fluiddicht abgeschlossen sind; dies gilt insbesondere für die Anordnung des Abstandshalters 56 und der Abdeckung 60, bei denen beispielsweise kraftschlüssig und/oder formschlüssig Fluiddichtheit, auch unter Verwendung von Dichtkomponenten, kraftschlüssig und/oder formschlüssig erreicht werden kann.

Über die Fenster 58 kann visuell das Fluidniveau in den jeweiligen Kontrollkammern 62 und damit in entsprechenden Probenaufnahmen 26 kontrolliert werden. Die Kontrollkammer 62, die Fenster 58, die ersten und zweiten Durchgangsöffnungen 46 und 50 und die Abdeckung 60 sind Teile von Niveauerfassungseinrichtungen für entsprechende Probenaufnahmen 26.

Alternativ oder ergänzend können einzelnen, mehreren oder allen Probenaufnahmen 26 jeweils ein oder mehrere Messsensoren zugeordnet sein, mit denen aktuelle Fluidniveaus darin erfasst werden können. Die Messsensoren können an den Innenseiten von Probenaufnahme 26 angebracht und/oder in diese integriert sein. Steuer- und Versorgungsverbindungen mit Messsensoren können drahtlos und/oder drahtgebunden sein.

Zur korrekt positionierten Anordnung des Begasungskopfes 12 an dem Probenaufnahmenblock 8 sind einzelne oder mehrere Führungen vorgesehen, die auch kodiert sein können. Die gezeigten Ausführungen umfassen sich von der Oberseite des Probenaufnahmenblocks 8 erstreckende Stifte 64, die beim Zusammenbau in Fühnmgsöffnungen 66 im Begasungskopf 12 eingreifen.

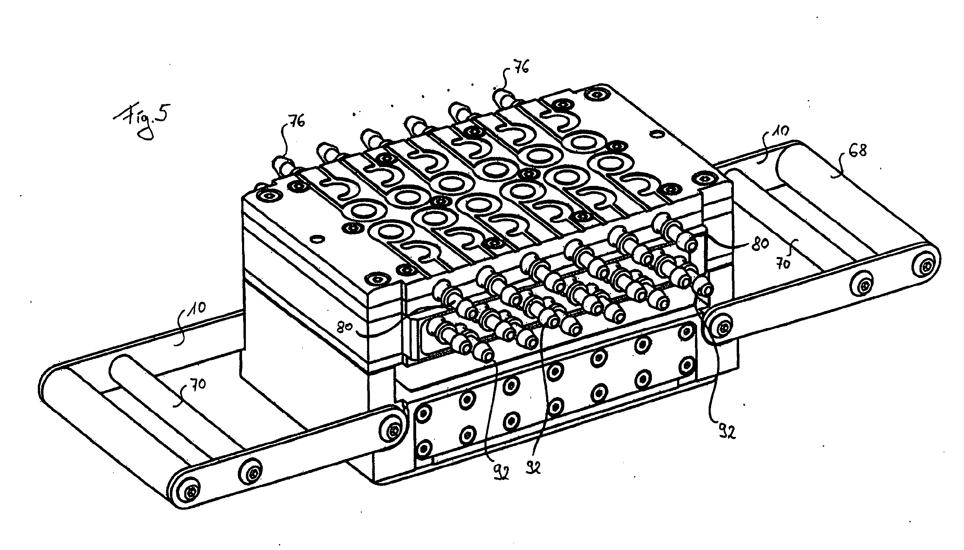

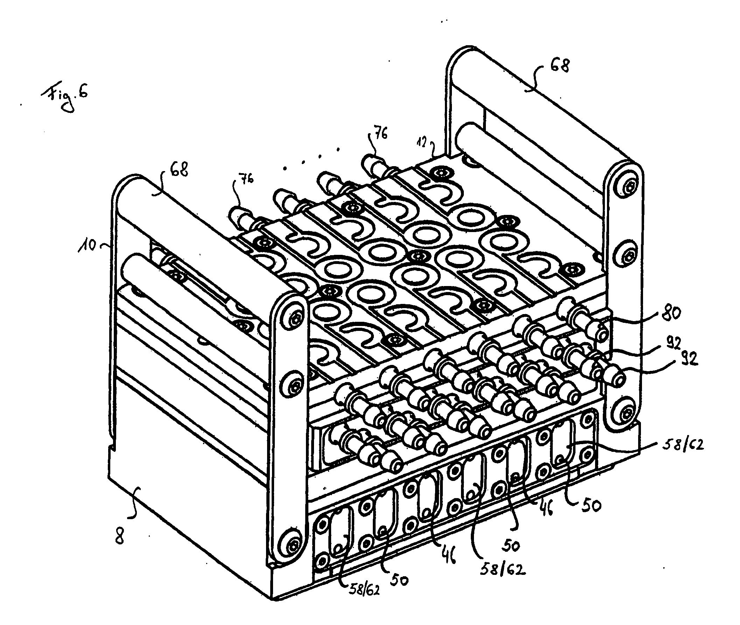

Ist, wie beispielsweise in Fig. 5 gezeigt, der Begasungskopf 12 an dem Probenaufnahmenblock 8 angeordnet, können diese durch Verschwenken der Hebel 10 aus der in Fig. 5 gezeigten Stellung in die in Fig. 6 gezeigte Stellung aneinander gesichert werden. Zum Entfernen des Begasungskopf 12 werden die Hebel 10 in die in Fig. 5 gezeigte Stellung bewegt.

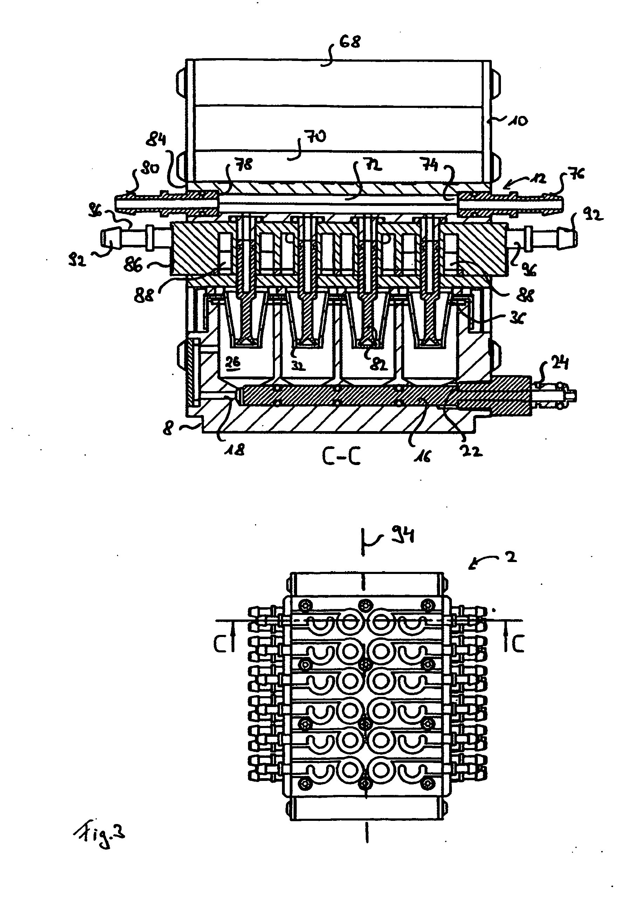

Die Hebel 10 können für deren Betätigung Griffe 68 aufweisen. Die Hebel 10 können Klemmelemente 70 umfassen, die bei der in Fig. 3 gezeigten Stellung mit Oberseiten (nicht bezeichnet) des Begasungskopfs 12 zusammenwirken und diesen an den Probenaufnahmenblock 8 drücken. Dies kann wenigstens dazu beitragen fluiddichte Verbindungsbereiche zwischen dem Begasungskopf 12 und dem Probenaufnahmenblock 8, insbesondere in Bereichen der Probenaufnahmen 26, zu erreichen.

Durch den Begasungskopf 12 erstrecken sich Begasungsmediumführungskanäle 72. Die Enden 74 der Begasungsmediumführungskanäle 72 sind jeweils mit einem Einlassanschluss 76 verbunden. Die gegenüberliegenden Enden 78 sind jeweils mit einem Auslassanschluss 80 verbunden. Die Einlassanschlüsse 76 sind jeweils vorgesehen, um Begasungsmedium zuzufügen. Über die Auslassanschlüsse 80 kann nicht benötigtes und/oder überschüssiges Begasungsmedium entfernt werden.

Die Begasungsmediumführungskanäle 72 sind jeweils den darunter liegenden Probenaufnahmen 26 zugeordnet. Gemäß den Darstellungen sind das jeweils die Probenaufnahmen, die in einer Reihe quer zur Längsrichtung des Probenaufnahmenblocks 8 angeordnet sind. Bei nicht gezeigten Ausführungsformen erstrecken sich die Begasungsmediumführungskanäle 72 in Längsrichtung des Probenaufnahmeblocks 8; auch hier können sie mit den jeweils darunter liegenden Probenaufnahmen 26 in Fluidverbindung stehen.

Es ist zu beachten, dass die Richtungen der Fluidkanäle 16 und die Begasungsmediumführungskanäle nicht übereinstimmen müssen, sondern z.B. senkrecht oder in einem anderen Winkel zueinander verlaufen können.

Die einem Begasungsmediumführungskanal 72 zugeordneten Probenaufnahmen 26 können mit dem gleichen Begasungsmedium versorgt werden, das sich von dem oder den Begasungsmedien unterscheiden kann, das bzw. die über entsprechende Begasungsmediumführungskanäle 72 anderen Probenaufnahmen 26 zugeführt werden kann.

Der Begasungskopf 12 weist für jede Probenaufnahme 26 einen Begasungsmediumauslass 82 auf. Abweichend von den Darstellungen können sich die Begasungsmediumauslässe 82 im zusammengebauten Zustand unterschiedlich weit in die jeweiligen Probenaufnahmen 26 erstrecken. Die Begasungsmediumauslässe 82 sind jeweils mit dem jeweiligen Begasungsmediumführungskanal 72 verbunden.

Darstellungsgemäß umfasst der Begasungskopf 12 einen oberen Teil 84, in dem die Begasungsmediumführungskanäle 72 ausgebildet ist. Der Begasungskopf 12 umfasst einen im zusammengebauten Zustand darunter angeordneten Teil 86, in dem die Begasungsmediumauslässe 82 angeordnet sind. Zur Verbindung der Begasungsmediumkanäle 72 mit den Begasungsmediumauslässen 82 sind Abgänge (nicht bezeichnet) an den Begasungsmediumkanälen 72 angeordnet. Die Abgänge sind im zusammengebauten Zustand mit den darstellungsgemäß oberen Enden der entsprechenden Begasungsmediumauslässe 82 verbunden. Um diese Verbindungsbereiche abzudichten, kann eine zwischen den Teilen 84 und 86 angeordnete Dichtung verwendet werden. Die Dichtung kann beispielsweise ringförmige einzelne Dichtelemente umfassen oder als flächige Dichtung mit Öffnungen ausgebildet sein.

Bei nicht gezeigten Ausführungsformen kann wenigstens für die Teile 84 und 86 ein einstückiger Aufbau verwendet werden, der baueinheitlich integriert Verbindungen zwischen den Begasungsmediumkanälen 72 und den Begasungsmediumauslässen 82 bereitstellt.

Vor einzelnen, mehreren oder allen Begasungsmediumauslässen 82 oder in diese integriert können bei nicht gezeigten Ausführungsformen Schließglieder angeordnet sein. Dies ermöglicht eine individuelle Steuerung der Verbindungen zwischen Begasungsmediumkanälen 72 und Begasungsmediumauslässen 82.

Auf diese Weise können Probenaufnahmen 26 einzeln, in Reihen, in Gruppen etc. gesteuert in Fluidverbindung mit dem jeweiligen Begasungsmediumführungskanal 72 gebracht und davon getrennt werden, um gesteuert die Zufuhr des Begasungsmediums zuzulassen bzw. zu verhindern. Derartige Ausführungsformen können in Verbindung mit oder ohne Verwendung von weiter unten beschriebenen Ausführungen, bei denen an Probenaufnahmen individuell Unterdruck angelegt werden kann, zur Begasung von Probenaufnahmen 26 bzw. den angeordneten Proben in beliebiger Zusammenstellung verwendet werden.

Den Begasungsmediumauslässen 82 ist jeweils eine Unterdruckkammer 88 zugeordnet. Die Unterdruckkammern 88 weisen jeweils wenigstens eine Öffnung 90 auf, die in die entsprechenden Probenaufnahme 26 mündet und über diese mit dem zugeordneten Begasungsmediumauslass 82 in Fluidverbindung steht. Jede Unterdruckkammer 88 ist über einen eigenen Unterdruckkanal 96 und einen damit verbundenen aus dem Probenaufnahmenblock 8 ragenden Unterdruckanschluss 92 mit einer Unterdruck erzeugenden Vorrichtung verbindbar. Die Unterdruckkammern können über eine gemeinsame Unterdruck erzeugende Einrichtung oder jeweils separat zugeordnete, individuelle Unterdruckquellen mit Unterdruck versorgt werden. Eine individuelle Unterdrucksteuerung für die Unterdruckkammern 88 kann auch durch zwischen Unterdruckquelle und Unterdruckkammer angeordnete Schließglieder (nicht gezeigt), beispielsweise in Form von Ventilen erreicht werden.

Bei den gezeigten Ausführungsformen sind die bezüglich der zentralen Ebene 94 auf einer Seite angeordneten Probenaufnahmen 26 Unterdruckkanälen 96 zugeordnet, die zu Unterdruckanschlüssen 92 auf der gleichen Seite führen. Bei weiteren Ausführungen können alle Unterdruckkanäle 96 zu einer Seite des Probenaufnahmeblocks 8 geführt sein. Wie in den Figuren gezeigt, sind die Unterdruckkanäle 96 bezüglich der horizontalen in unterschiedlich, hier zwei, Ebenen und/oder versetzt zueinander, angeordnet. Das erlaubt eine kompaktere Bauform verglichen mit einer Anordnung in einer Ebene. Für eine kompakte Bauform können - ergänzend oder alternativ - Unterdruckkanäle 96 verwendet werden, die sich nicht geradlinig zwischen Unterdruckanschluss und Unterdruckkammer erstrecken, sondern sich wenigstens teilweise gekrümmt zwischen Unterdruckkammern und Unterdruckanschluss erstrecken.

Es können auch mehreren Probenaufnahmen 26 einzelne oder mehrere gemeinsame Unterdruckkammern zugeordnet sein, die über wenigstens eine Öffnung zu jeder Probenaufnahme 26 verfügen. Auch eine einzelne Unterdruckkammer für alle Probenaufnahmen 26 ist vorgesehen. In solchen Fällen können die einer gemeinsamen Unterdruckkammer 88 zugeordneten Probenaufnahmen 26 gemeinsam mit Unterdruck beaufschlagt werden. Eine individuelle Begasung von Probenaufnahmen 26 kann dann, wie oben ausgeführt, z.B. über auf Begasungsmediumauslässe 82 wirkende Schließglieder erreicht werden.

Die Basis 4 weist eine Aufnahme 98 auf, die zur Anordnung des Probenaufnahmenblocks 8 vorgesehen ist. Zur Positionierung des Probenaufnahmenblocks 8 an der Basis 4 kann der Probenaufnahmenblock 8 an seiner Unterseite komplementär zu der Aufnahme 98 bzw. den diese begrenzenden Ränder 100 ausgebildet sein.

Die Basis 4 kann zur Temperatursteuerung des Probenaufnahmenblocks 8 ausgeführt sein. Hierfür können insbesondere Bereiche der Basis 4, die bei darauf angeordnetem Probenaufnahmenblock 8 diesen kontaktieren, aus wärmeleitfähigem Material hergestellt sein. Bereiche der Basis 4 ohne Kontakt zum Probenaumahmenblock 8 können demgegenüber beispielsweise durch entsprechende Materialwahl und/oder Beschichtungen und/oder integrierte Strukturen ausgeführt sein, um unerwünschten Wärmeverlust und Wärmeabstrahlung zur Umgebung zu vermeiden. Die Basis 4 kann bei einer hier nicht gezeigten Ausführungsform einen oder mehrere Kanäle aufweisen, die mit Fluid gefüllt und/oder von Fluid durchströmt werden können, dessen Temperatur für eine gewünschte Temperatur des Probenaufnahmenblocks 8 sorgt.

Bei der gezeigten Ausführungsform der Basis 4 ist in dieser eine Aufnahme 102 ausgeformt, in die eine elektrisch erwärmbare Einrichtung 104, beispielsweise eine sogenannte Heizpatrone, eingebracht werden kann. Durch Steuerung der Temperatur der Vorrichtung 104 kann die Basis 4 und insbesondere deren Bereiche in Kontakt mit dem Probenaufnahmenbock 8 hinsichtlich der Temperatur so gesteuert werden, dass der Probenaufnahmenblock 8 eine gewünschte Temperatur erreicht. Als Wärmeeinrichtung kann z.B. eine Heizplatte verwendet werden.

Eine von der Basis 4 bereitgestellte Wärmesteuerung kann auch für den Begasungskopf 12 wirken. Hierfür können beispielsweise die Bereiche des Begasungskopfs 12 und des Probenaufnahmenblocks 8, die einander kontaktieren, wärmeleitfähig ausgestaltet sein.

Das Begasungssystem 2 kann als Systemkomponenten ferner folgende nicht dargestellte Komponenten umfassen: Eine Vorrichtung, mit der Fluid, beispielsweise Nährmedium für Zellkulturen, in die Fluidkanäle 16 eingebracht werden kann. Eine oder mehrere Einrichtungen, um in den Unterdruckkammern 86 Unterdruck zu erzeugen. Auch Mittel, beispielsweise Schläuche, Kupplungen, starre Leitungen und dergleichen, zum Anschluss der genannten Vorrichtungen können umfasst sein. Dies gilt auch für Komponenten zu deren Steuerungen.

Letztere können auch von der Steuerung 14 umfasst sein. Vereinfachend ist die Steuerung 14 für eine Ausführung dargestellt, bei der die Steuerung 14 zur Steuerung der wärmeerzeugenden Vorrichtung 104 dient. Neben Eingabemitteln (Knöpfen, Schieber, Tastaturen etc.) kann die Steuerung 14 Anzeigen aufweisen, die aktuelle Temperaturen der Basis 4, der wärmeabgebenden Einrichtung 104, des Probenaufnahmenblocks 8 und/oder des Begasungskopfs 12 anzeigen.

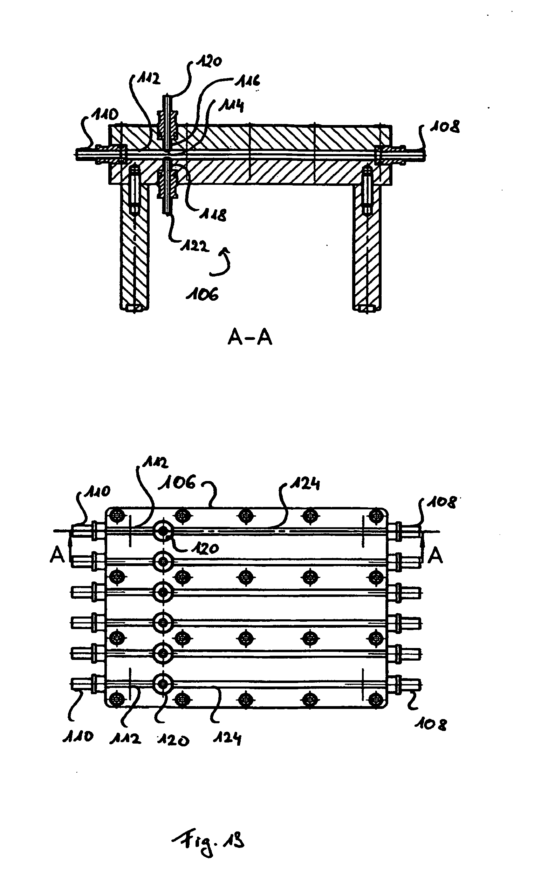

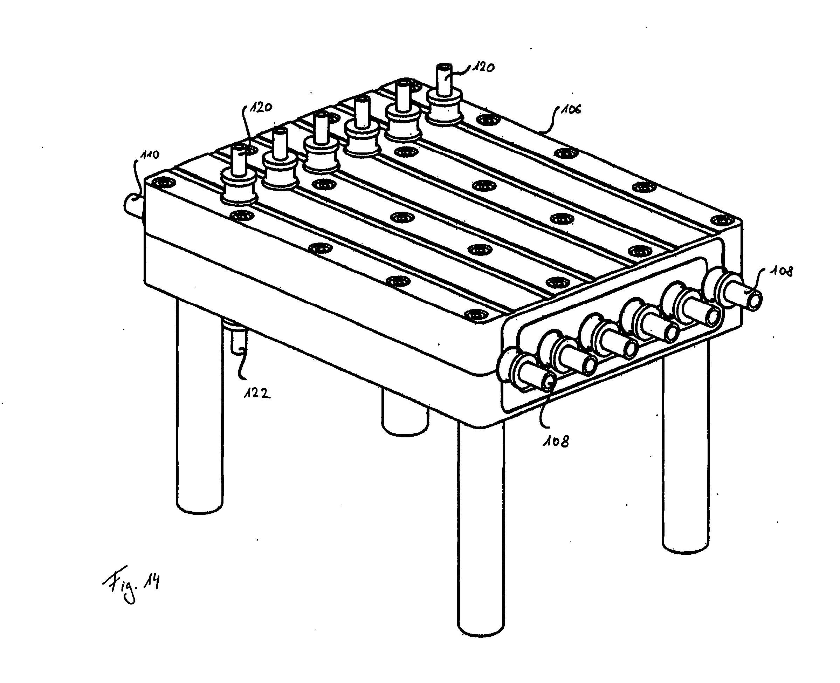

Um den Begasungsmediumfuhrungskanälen 72 und damit den Probenbehältern 26 Begasungsmedium zuzuführen, kann eine insbesondere in Fig. 13 und 14 veranschaulichte Mischeinrichtung 106 verwendet werden. Die Mischeinrichtung 106 weist bei den dargestellten Ausführungsformen für jeden Einlassanschluss 76 der Begasungsmediumführungskanäle 72 einen ersten Fluidauslass 108 und erste Fluideinlässe 110 in gleicher Anzahl auf. Ausgehend von den ersten Fluideinlässen 110 erstrecken sich Fluidkanäle 112 bis zum einem Mischbereich 114. In den Mischbereich 114 münden Fluidkanäle 116 und optionale Fluidkanäle 118, die andererseits mit zweiten Fluideinlässen 120 bzw. optionalen dritten Fluideinlässen 122 verbunden sind. Ausgehend von den Mischbereich 114 erstrecken sich jeweils Fluidkanäle 124 zu den entsprechenden ersten Fluidauslässen 108.

Die ersten Fluideinlässe 110 sind jeweils zur Verbindung mit einer Quelle (nicht gezeigt) für ein Fluid vorgesehen, das ein Bestandteil eines vorgesehenen Begasungsmediums sein soll, das über den entsprechenden ersten Fluidauslass 108 abgegeben werden kann. Es können für alle ersten Fluideinlässe 110 eine oder mehrere vergleichbare Fluidquellen oder für wenigstens zwei erste Fluideinlässen 110 unterschiedliche Fluidquellen verwendet werden.

Die zweiten Fluideinlässe 120 sind ebenfalls jeweils zur Verbindung mit einer Quelle (nicht gezeigt) für anderes Fluid vorgesehen, das ein Bestandteil eines vorgesehenen Begasungsmediums sein soll, das über den entsprechenden ersten Fluidauslass 108 abgegeben werden kann. Es können für alle zweiten Fluideinlässe 120 eine oder mehrere vergleichbare Fluidquellen oder für wenigstens zwei zweite Fluideinlässen 120 unterschiedliche Fluidquellen verwendet werden. Die Zufuhr unterschiedlicher Fluide in den Mischbereich erlaubt es Fluidmischung(en) zu erzeugen, die als Begasungsmedium über die ersten Fluidauslässe 108 abgegeben werden können.

Über die Fluidkanäle 112 und 116 zu dem entsprechenden Mischbereich 114 geführte Fluide können dort gemischt werden, um ein Fluid zu erhalten, dass als Begasungsmedium Probenaufnahmen 26 zugeführt werden soll. Über die optionalen dritten Fluideinlässe 122 kann weiteres Fluid dem Mischbereich zugeführt werden; insbesondere ist sind die dritten Fluideinlässe 122 zur Zufuhr eines Fluids vorgesehen, dass sich von über die zweiten Fluideinlässe 120 zuführbarem Fluid unterscheidet. So kann z.B. Luft zur weiteren Verdünnung über die dritten Fluideinlässe 122 eingebracht werden.

Der Begasungsvorrichtung zuzuführendes Begasungsmedium (z.B. eine Fluid eines Typs, eine Mischung von zwei oder mehr Fluiden) wird von dem Mischbereich 114 über die Fluidkanäle 124 und die ersten Fluidauslässe 108 bereitgestellt.

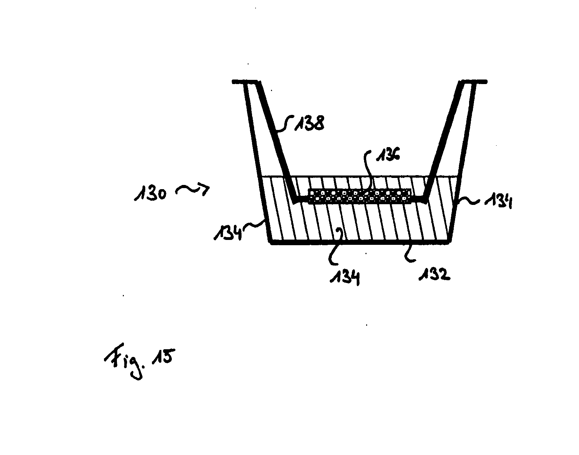

Die Probenaufnahmen 26 können, wie oben ausgeführt, jeweils zur Aufnahme eines einzelnen Probenbehälters ausgeführt sein. Mögliche Bauformen für Probenbehälter, die als einzelne, separate Probenbehälter oder als Einsatz baueinheitlich integrierte Probenbehälter verwendet werden können, sind unter Bezugnahme auf Fig. 15 erläutert. Ein Probenbehälter 130 weist einen Boden 132 und Seitenwände 134 auf. Der von dem Boden 132 und den Seitenwänden 134 begrenzte Innenraum des Probenbehälters 130 kann parallel zum Boden 132 einen kreisförmigen Querschnitt aufweisen. In diesem Innenraum kann eine zu begasende Probe unmittelbar, z. B. am Boden 132, angeordnet werden.

Die zu begasende Probe kann zusammen mit einem Fluid 134 (z. B. Nährlösung) im Probenbehälter angeordnet sein. In solchen Fällen kann die zu begasende Probe (teilweise) in das Fluid 134 eingetaucht sein, dieses nur kontaktieren oder von diesem getrennt angeordnet sein. Fluid 134 kann beispielsweise über die Fluidkanäle 16 zugeführt und/oder entfernt werden.

Ferner kann eine Membran 136, z. B. eine mikroporöse Membran, verwendet werden, auf der zu untersuchende Proben, beispielsweise Zellen, angeordnet werden bzw. sein können. Die Membran 136 kann - wenn vorhanden - (teilweise) in das Medium 134 eingetaucht sein, dieses nur kontaktieren oder von diesem getrennt angeordnet sein. Die mikroporöse Membran 136 kann beispielsweise mittels eines Probenbehältereinsatzes 138 angeordnet werden. Auf der Membran 136 können zu untersuchende Proben, beispielsweise Zellen, angeordnet werden bzw. sein.

Der Betrieb der oben beschriebenen Ausführungsformen kann wie folgt sein.

Im Folgenden wird vereinfacht auf eine Begasungsvorrichtung bzw. eine Begasungsvorrichtung umfassendes Begasungssystem Bezug genommen. Die folgenden Ausführungen geltend entsprechend bei Verwendung mehrerer Begasungsvorrichtungen.

In einzelnen, mehreren oder allen Probenaufnahmen 26 des Probenaufnahmenblocks 8 werden zu begasende Proben eingebracht. Mögliche Proben umfassen Zellkulturen, Zellproben, Gewebeproben, Bakterien, Pilze und dergleichen. Zur Begasung mögliche Begasungsmedien umfassen gasförmige Medien beispielsweise in Form eines reinen Gases oder Gase, Gasgemische, Aerosole, zerstäubte Flüssigkeiten, Gase oder Gasmischungen mit Flüssigkeitströpfchen, Schwebeteilchen, Feststoffpartikeln, gasförmige Suspensionen, zerstäubte Suspensionen usw. Begasungsmedium kann beispielsweise von einem sogenannten Rauchroboter bereitgestellt werden, mit dem auf technische Weise Rauch von Zigaretten, vergleichbar wie beim Zigarettenrauchen, gewonnen werden kann. Ferner können als Begasungsmediumquellen sogenannte Aerosolgeneratoren und/oder Partikelverdichter dienen, die (Umwelt)Atmosphären konzentrieren können.

Die Bestückung von Probenaufnahmen 26 mit Proben kann beispielsweise in einer speziellen Umgebung (z. B. Reinraumumgebung) erfolgen. Dies wird durch die Verwendung einer erfindungsgemäßen Begasungsvorrichtung erleichtert. Diese kann separat in eine solche Umgebung gebracht werden. Komponenten, die (nur) beim Begasungsvorgang benötigt werden, wie zum Beispiel Begasungsmedien bereitstellende Vorrichtungen, Steuerungen, Halterungen etc. müssen dabei nicht mit bewegt werden.

Nach Anordnen von Proben in Probenbehältern 26 werden der Begasungsmediumblock 8 und der Begasungskopf 12 aneinander gesichert. Die Begasungsvorrichtung wird dann an der Basis 4, die beispielsweise in einem anderen Raum vorhanden sein kann, angeordnet. Dort werden die Zu- und Ableitungen, Fluidanschlüsse, Unterdruckanschlüsse etc. der Begasungsvorrichtung angeschlossen. Ausführungsabhängig kann einzelnen, mehreren oder allen Probenbehältern 26 beispielsweise über die Fluidkanäle 16. Für jede Probenaufnahme 26 ist es mittels der jeweiligen Niveauerfassungseinrichtung möglich, den aktuellen Fluidpegel in der Probenaufnahme 26 zu ermitteln bzw. zu überwachen. Informationen über Fluidniveaus in Aufnahmen 26 können zur Steuerung der Zufuhr und/oder Abfuhr von Fluid von bzw. zu einzelnen, mehreren oder allen Probenbehältern 26 verwendet werden.

Die für einzelne, mehrere oder alle Begasungsmediumauslässe 82 steuerbare Fluidzufuhr kann bezüglich eines Begasungsmediumauslasses 82 durch zwei alternativ oder gemeinsam durchführbare Maßnahmen erreicht werden. Die Steuerung von Begasungsmedium für einen Begasungsmediumauslass 82 kann durch Steuerung von dem jeweiligen Begasungsmediumauslass 82 zugeführtem Begasungsmedium erreicht werden. Hierfür kann die einem entsprechenden Begasungsmediumkanal 72 zugeführte Begasungsmenge beispielsweise stromauf des Einlassanschlusses 76 gesteuert werden. Die zur Verfügung stehende Menge an Begasungsmedium kann durch ein zwischen Begasungsmediumkanälen 72 und dem offenen, unteren Ende des Begasungsmediumauslass 82 gesteuert werden; das Schließglied, beispielsweise in Form eines Ventils, kann im oder am Begasungsmediumauslass 82 angeordnet sein.

Eine weitere Vorgehensweise zur Begasungsmediumsteuerung kann über die Unterdruckkammern 88 erreicht werden. Legt man an den entsprechenden Unterdruckanschluss 92 Unterdruck an, wird über die Unterdruckkammer 88 und deren wenigstens eine Öffnung 90 Unterdruck in der zugeordneten Probenaufnahme 26 erzeugt. Dieser Unterdruck wirkt über das untere, offene Ende des jeweiligen Begasungsmediumauslass 82 auf Begasungsmedium. Das Begasungsmedium wird von diesem Unterdruck in die Probenaufnahme 26 befördert und kann dort eine Probe begasen.

Die vorliegende Erfindung sorgt für einen modularen Aufbau von Begasungssystemen und Vorrichtungen. Die trennbare Ausführung der Begasungsvorrichtungen innerhalb des Begasungssystems erlaubt es, die jeweiligen Arbeits- und Betriebsschritte an unterschiedlichen Stellen/Orten durchzuführen. Die Begasungssysteme können nacheinander mit einzelnen oder mehreren auch unterschiedlichen Begasungsvorrichtungen verwendet werden, wobei im übrigen die gleichen Komponenten (im Wesentlichen) ohne Änderung weiter benutzt werden können. Dies gilt insbesondere für Fluid- und Begasungsmediumzuführungen, -anschlüsse und dergleichen. Auch die Basis und Steuerkomponenten können beibehalten werden. Es können Begasungsvorrichtungen mit unterschiedlicher Anzahl an Probenaufnahmen ohne weitere Änderungen im Gesamtsystem verwendet werden. Die Konstruktionen insbesondere des Probenaufnahmenblocks sind aufgrund von zum Teil einstückigen Ausführungen einfach, automatisiert herzustellen, robust und einfach zu handhaben. Insbesondere für experimentelle Laboranwendungen, aufwändige Verbindungsleitung umfassende Anordnungen mit zum Teil zahlreichen aus zerbrechlichen Materialien (z. B. Glas) hergestellten Komponenten werden vermieden.

## Patentansprüche

1. Begasungsvorrichtung, mit:
- einem einstückigen Probenaufnahmenblock (8), der darin ausgebildete Probenaufnahmen (26) und eine darin ausgebildete Fluidversorgungseinrichtung (16) für die Probenaufnahmen (26) aufweist, und
- einem Begasungskopf (12) zur Zufuhr von Begasungsmedium, der mit dem Probenaufnahmenblock (8) verbunden ist, für jede der Probenaufnahmen (26) einen Begasungsmediumauslass (82) aufweist und eine darin ausgebildete Einrichtung zur Begasungsmediumführung (72) zu den Begasungsmediumauslässen (82) aufweist,
**dadurch gekennzeichnet, dass**
- die Probenaufnahmen (26) in mehreren Reihen angeordnet sind, und
- den Probenaufnahmen (26) jeweils eine dem entsprechenden Begasungsmediumauslass (82) zugeordnete und im Probenaufnahmenblock (8) angeordnete Unterdruckkammer (88) zugeordnet ist und eine Einrichtung zur individuellen Unterdrucksteuerung der Unterdruckkammern (88) vorgesehen ist, um den Probenaufnahmen (26) separat, unabhängig von einer Begasungsmediumzufuhr zu anderen Probenaufnahmen (26) Begasungsmedium zuzuführen.

2. Begasungsvorrichtung nach Patentanspruch 1, bei der die Einrichtung zur individuellen Unterdrucksteuerung für jede Unterdruckkammer (88) einen Unterdruckkanal (96) und einen damit verbundenen Unterdruckanschluss (92) jeweils zu Verbindung mit einer Unterdruck erzeugenden individuellen Unterdruckquelle aufweist.

3. Begasungsvorrichtung nach Patentanspruch 1, bei der die Einrichtung zur individuellen Unterdrucksteuerung für jede Unterdruckkammer (88) einen Unterdruckkanal (96) und einen damit verbundenen Unterdruckanschluss (92) zu Verbindung mit einer Unterdruck erzeugenden gemeinsamen Unterdruckquelle aufweist und zwischen den Unterdruckkammern (88) und der gemeinsamen Unterdruckquelle jeweils ein Schließglied angeordnet ist.

4. Begasungsvorrichtung nach einem der vorherigen Patentansprüche, bei der den Probenaufnahmen (26) jeweils eine Niveauerfassungseinrichtung (46, 50, 58, 60, 62) zugeordnet ist.

5. Begasungsvorrichtung nach Anspruch 4, bei der die Niveauerfassungseinrichtungen (46, 50, 58, 60, 62) jeweils eine an einer Außenseite des Probenaufnahmenblocks (8) individuelle Niveauanzeige (46, 50, 58, 60, 62) umfassen.

6. Begasungsvorrichtung nach Anspruch 5, bei der der Begasungskopf (12) für die Probenaufnahmen (26) jeweils eine Ansaugöffnung umfasst, die zu der entsprechenden Unterdruckkammer (88) führt.

7. Begasungsvorrichtung nach einem der vorherigen Ansprüche, mit einer zur Anordnung auf einer Oberseite des Probenaufnahmenblocks (8) vorgesehenen Dichtung.

8. Begasungsvorrichtung nach Anspruch 7, bei der die Dichtung Öffnungen aufweist, durch die Probenbehälter wenigstens teilweise in die Probenaufnahmen (26) einbringbar sind.

9. Begasungsvorrichtung nach einem der vorherigen Patentansprüche, mit einem Einsatz (36), der baueinheitlich integriert eine Anzahl an Probenbehältern (32) aufweist, die der Anzahl an Probenaufnahmen (26) entspricht oder kleiner ist, und an seiner Unterseite in entsprechende Ausnehmungen an den oberen offenen Enden der Probenaufnahmen (26) eingreifende Nasen (38) aufweist.

10. Begasungssystem mit
- wenigstens einer Begasungsvorrichtung nach einem der Ansprüche 1 bis 9, und
- einer Basis (4) zur Anordnung des Probeaufnahmeblocks.

11. Begasungssystem nach Anspruch 10, ferner mit einer Vorrichtung zur Mischung von Begasungsmedien und Zufuhr von gewünschtem Begasungsmedium zu den Probenaufnahmen (26).

## Claims

1. A gassing apparatus comprising:
- an integral sample receiving block (8) including sample receptacles (26) formed therein and a fluid supply device (16) formed therein for the sample receptacles (26), and
- a gassing head (12) for supplying gassing medium being connected to the sample receiving block (8), including a gassing medium outlet (82) for each of the sample receptacles (26), and including a device (72) formed therein for directing gassing medium to the gassing medium outlets (82),
**characterized in that**
- the sample receptacles (26) are arranged in a plurality of rows, and
- the sample receptacles (26) are each associated with a vacuum chamber (88) being associated with the corresponding gassing medium outlet (82) and arranged within the sample receiving block (8), and a device for individual vacuum control of the vacuum chambers (88) is provided for separately supplying the sample receptacles (26) with gassing medium independently of a gassing medium supply to the other sample receptacles (26).

2. The gassing apparatus according to patent claim 1, wherein the device for individual vacuum control for each vacuum chamber (88) includes a vacuum channel (96) and a vacuum connection (92) connected thereto each adapted for connection to a vacuum-generating individual vacuum source.

3. The gassing apparatus according to patent claim 1, wherein the device for individual vacuum control for each vacuum chamber (88) includes a vacuum channel (96) and a vacuum connection (92) connected thereto adapted for connection to a vacuum-generating common vacuum source, and wherein a closure member is arranged between the vacuum chambers (88) and the common vacuum source in each case.

4. The gassing apparatus according to any one of the preceding claims, wherein the sample receptacles (26) are each associated with a level detection device (46, 50, 58, 60, 62).

5. The gassing apparatus according to claim 4, wherein the level detecting devices (46, 50, 58, 60, 62) each comprise an individual level indicator (46, 50, 58, 60, 62) located at the outside of the sample receiving block (8).

6. The gassing apparatus according to claim 5, wherein the gassing head (12) for the sample receptacles (26) comprises a suction opening leading to the corresponding vacuum chamber (88) in each case.

7. The gassing apparatus according to any one of the preceding claims, comprising a sealing adapted for arrangement on an top side of the sample receiving block (8).

8. The gassing apparatus according to claim 7, wherein the sealing comprises openings through which the sample containers are at least partially insertable into the sample receptacles (26).

9. The gassing apparatus according to any one of the preceding claims, comprising an insert (36) including a number of sample containers (32) integrated in the manner of a modular unit, said number corresponding to or being less than the number of sample receptacles (26), and including, at its bottom side, projections (38) engaging with corresponding recesses at the top open ends of the sample receptacles (26).

10. A gassing system comprising:
- at least one gassing apparatus according to any one of claims 1 to 9, and
- a base (4) adapted for arrangement of the sample receiving block.

11. The gassing system according to claim 10, further comprising a device for mixing gassing media and for supplying desired gassing medium to the sample receptacles (26).

## Revendications

1. Dispositif de gazage, comprenant :
- un bloc de réception d'échantillons (8) d'une seule pièce, qui comporte des réceptacles d'échantillons (26) formés dans celui-ci et un moyen d'alimentation en fluide (16) formé dans celui-ci pour les réceptacles d'échantillons (26), et
- une tête de gazage (12) servant à amener un agent de gazage, qui est reliée au bloc de réception d'échantillons (8), comporte une sortie d'agent de gazage (82) pour chacun des réceptacles d'échantillons (26) et comporte, formé dans celle-ci, un moyen de guidage d'agent de gazage (72) vers les sorties d'agent de gazage (82),
**caractérisé en ce que**
- les réceptacles d'échantillons (26) sont disposés en plusieurs rangées, et
- une chambre à dépression (88), associée à la sortie d'agent de gazage (82) correspondante et disposée dans le bloc de réception d'échantillons (8), est associée à chacun des réceptacles d'échantillons (26) et un moyen de commande individuelle de dépression des chambres à dépression (88) est prévu pour amener de l'agent de gazage aux réceptacles d'échantillons (26) d'une manière séparée, indépendante d'une amenée d'agent de gazage à d'autres réceptacles d'échantillons (26).

2. Dispositif de gazage selon la revendication 1, dans lequel le moyen de commande individuelle de dépression comporte, pour chaque chambre à dépression (88), un canal de dépression (96) et un raccord de dépression (92) relié à celui-ci pour la liaison respective à une source de dépression individuelle générant une dépression.

3. Dispositif de gazage selon la revendication 1, dans lequel le moyen de commande individuelle de dépression comporte, pour chaque chambre à dépression (88), un canal de dépression (96) et un raccord de dépression (92) relié à celui-ci pour la liaison à une source de dépression commune générant une dépression, et un organe de fermeture est disposé chaque fois entre les chambres à dépression (88) et la source de dépression commune.

4. Dispositif de gazage selon une des revendications précédentes, dans lequel un moyen de détection de niveau (46, 50, 58, 60, 62) est associé à chacun des réceptacles d'échantillons (26).

5. Dispositif de gazage selon la revendication 4, dans lequel les moyens de détection de niveau (46, 50, 58, 60, 62) comprennent chaque fois une indication de niveau (46, 50, 58, 60, 62) individuelle sur une face extérieure du bloc de réception d'échantillons (8).

6. Dispositif de gazage selon la revendication 5, dans lequel la tête de gazage (12) pour les réceptacles d'échantillons (26) comprend chaque fois une ouverture d'aspiration qui conduit à la chambre à dépression (88) correspondante.

7. Dispositif de gazage selon une des revendications précédentes, avec un joint d'étanchéité prévu pour être disposé sur une face supérieure du bloc de réception d'échantillons (8).

8. Dispositif de gazage selon la revendication 7, dans lequel le joint d'étanchéité comporte des ouvertures à travers lesquelles des récipients à échantillons peuvent être introduits au moins partiellement dans les réceptacles d'échantillons (26).

9. Dispositif de gazage selon une des revendications précédentes, avec un insert (36) qui comporte une pluralité de récipients à échantillons (32) intégrés de manière à former une unité de montage, dont le nombre correspond à celui des réceptacles d'échantillons (26) ou est plus petit, et qui comporte, sur sa face inférieure, des ergots (38) qui s'engagent dans des évidements correspondants aux extrémités supérieures ouvertes des réceptacles d'échantillons (26).

10. Système de gazage comprenant
- au moins un dispositif de gazage selon une des revendications 1 à 9, et
- une base (4) pour l'agencement du bloc de réception d'échantillons.

11. Système de gazage selon la revendication 10, comprenant en outre un dispositif pour mélanger des agents de gazage et amener l'agent de gazage souhaité aux réceptacles d'échantillons (26).
